## Europäisches Patentamt

(19) **European Patent Office**

**Office européen des brevets**

(11) Veröffentlichungsnummer: **0 016 338**
**B1**

(12) **EUROPÄISCHE PATENTSCHRIFT**

(45) Veröffentlichungstag der Patentschrift:
**27.10.82**

(21) Anmeldenummer: **80100776.6**

(22) Anmeldetag: **15.02.80**

(51) Int. Cl.³: **A 61 B 17/18**

(54) Spannvorrichtung für eine Druckplatte zur Durchführung einer Druckosteosynthese.

(30) Priorität: **23.03.79 DE 2911386**
**21.09.79 DE 2938202**

(43) Veröffentlichungstag der Anmeldung:
**01.10.80 Patentblatt 80/20**

(45) Bekanntmachung des Hinweises auf die Patenterteilung:
**27.10.82 Patentblatt 82/43**

(84) Benannte Vertragsstaaten:
**AT BE CH DE FR GB IT NL SE**

(56) Entgegenhaltungen:
**DE-A-2 802 090**
**DE-B-1 791 228**
**FR-A-2 210 908**
**FR-A-2 289 153**
**US-A-3 386 437**
**US-A-3 528 085**
**US-A-3 534 731**

(73) Patentinhaber: **Brinckmann, Paul, Prof. Dr.,**
**Rinscheweg 3, D-4400 Münster (DE)**

(72) Erfinder: **Brinckmann, Paul, Prof. Dr., Rinscheweg 3,**
**D-4400 Münster (DE)**

(74) Vertreter: **Habbel, Hans-Georg, Dipl.-Ing.,**
**Postfach 3429 Am Kanonengraben 11, D-4400 Münster**
**(DE)**

Spannvorrichtung für eine Druckplatte zur Durchführung einer Druckosteosynthese

Die Erfindung bezieht sich auf eine Spannvorrichtung für eine Druckplatte zur Durchführung einer Druckosteosynthese gemäß dem Oberbegriff des Hauptanspruches.

Die Entwicklung stabiler Osteosyntheseverfahren hat zur sogenannten Druckosteosynthese geführt. Die maßgeblichen Vorteile dieser übungsstabilen Druckosteosynthese liegen neben der guten Fragmentreposition vor allem in der Möglichkeit der Frühmobilisierung mit Vermeidung langer Bettlägerigkeit, funktionellen Durchblutungsstörungen, Inaktivitätsatrophie von Muskeln und Knochen sowie Gelenkkontrakturen. Durch die Kompression der Knochenfragmente wird eine bessere mechanische Stabilität als bei einfacher Adaptionsosteosynthese erreicht, was nach derzeit herrschender Meinung der entscheidende Vorteil der Druckosteosynthese ist. Die Verhinderung jeglicher Relativbewegung der gegeneinander verspannten Fragmente ist die Voraussetzung für die sogenannte Primärheilung.

Zur Durchführung der Druckosteosynthese sind in der Praxis im wesentlichen drei Verfahren bekanntgeworden.

Bei der sogenannten Danis-Platte erfolgt der Kompressionsvorgang durch eine in Plattenlängsachse ausgerichtete Kompressionsschraube, die auf eine die Platte am Knochen festlegende Befestigungsschraube wirkt. Bei der sogenannten AO-Kompressionsplatte mit abnehmbarem Plattenspanner wird an der eigentlichen Druckplatte ein Zugelement des Plattenspanners festgelegt, während das Druckelement über eine zusätzliche Befestigungsschraube am Knochen gelagert werden muß.

Bei der erstgenannten Einrichtung ist es nachteilig, daß die Plattenstärke sehr groß sein muß.

Beiden bekannten Einrichtungen haftet der Nachteil an, daß die Wunde über die Länge der eigentlichen Druckosteosyntheseplatte hinaus verlängert und der Knochen freigelegt werden muß, was schwierig und in Gelenknähe häufig gar nicht möglich ist. Der Knochen wird bei dem abnehmbaren Plattenspanner durch ein zusätzliches Loch verletzt. Außerdem ist die Arbeitsrichtung in Richtung der Längsachse der Druckplatte gerichtet, so daß hierdurch die Wundränder beeinflußt werden können.

Bei den einfachen, sogenannten selbstspannenden Druckplatten wird ein Handwerkerprinzip eingesetzt, das sich durch eine sphärich gestaltete Schraube kennzeichnet, die mit einem ovalen, eine entsprechende Randneigung aufweisenden Schraubenloch zusammenwirkt. Diese Anordnung hat den Vorteil, daß eine Vergrößerung der Wunde über die Druckplatte hinaus nicht erforderlich ist und daß die eigentliche Spanneinrichtung senkrecht von oben — von der Druckplatte aus gesehen — betätigt werden kann. Nachteil dieser selbstspannenden Druckplatte ist der relativ kurze Spannweg, der um so kürzer wird, je dünner die Platte gehalten wird oder gehalten werden muß.

In der US-A-3 528 085 wird eine derartige Spanneinrichtung beschrieben, bei der der Spannweg dadurch vergrößert wird, daß die Ränder der Schraublöcher relativ dick ausgebildet werden. Bei vielen Einsatzfällen ist ein Schließen der Wundränder über eine solche dicke Platte hinweg nicht möglich, so daß der Einsatz solcher Platten außerordentlich beschränkt ist.

Die selbstspannende Druckplatte hat zudem den Nachteil, daß ggf. durch die Druckeinwirkung zwischen Spannschraube und Platte Beschädigungen der Oberfläche der Druckplatte entstehen, so daß hier erhöhte Oberflächenkorrosionen der Implantate und dadurch verursachte Schädigungen des umgebenden Gewebes eintreten können.

Aus der DE-A-2 802 090 ist ein Kompressions-/Distraktionsgerät für die Osteosynthese bekannt gemäß dem Oberbegriff des Hauptanspruches, bei welchem die eigentliche Spannvorrichtung auf der Oberseite der Druckplatte über einem in der Druckplatte vorgesehenen Langloch angeordnet ist. Die Vorrichtung stützt sich dabei über ein hakenförmiges Teil an dem äußeren Längsrand des Langloches ab. Eine ortsfeste Festlegung der Spannvorrichtung erfolgt dabei nicht, sondern das hakenförmige Teil wirkt nur hinsichtlich der Druckaufnahme in Längsrichtung der Druckplatte. Bewegungen nach oben oder seitliche Bewegungen sind bei dieser Einrichtung möglich, so daß kein sicheres Arbeiten möglich ist, insbesondere sind quer zur Ebene der Druckplatte gerichtete Kippbewegungen möglich. Der Einsatz dieser bekannten Vorrichtung erfordert außerordentliche Aufmerksamkeit, was bei schwierigen Operationen nicht zu vertreten ist.

Der Erfindung liegt die Aufgabe zugrunde, eine Spannvorrichtung der vorstehend genannten Art für die Druckosteosynthese zu schaffen, bei welcher unter Beibehaltung der Vorteile, daß die Spannbewegung ohne Zugbelastung des die Vorrichtung mit dem betreffenden Knochenfragment verbindenden Verbindungsmittels erfolgt, große Spannweiten sowie große Spannkräfte erreicht werden können, ein zusätzliches Erweitern der Wunde nicht erforderlich ist und keine Beschädigungen der Platte eintreten können, die Spannvorrichtung derart ortsfest an der Druckplatte anbringbar ist, daß ein einfaches Arbeiten mit der Vorrichtung in allen Lagen der Druckplatte möglich ist.

Diese Aufgabe wird erfindungsgemäß durch die im kennzeichnenden Teil des Hauptanspruches angegebenen Maßnahmen gelöst. Die Ansprüche 2 bis 7 enthalten zweckmäßige Ausführungsformen der Erfindung.

Durch die Erfindung wird ein Hilfsmittel geschaffen, bei dem die Wunde über die Länge der Druckplatte hinaus nicht geöffnet werden muß. Spannstrecken zwischen 12 und 15 mm sind leicht und unabhängig von der Stärke der Druckplatte realisierbar. Ein zusätzliches Schraubenloch im Knochen für den Spanner wird vermieden. Durch die Möglichkeit, eine 45° geneigte Gleitebene im Spannblock auszuarbeiten, wird eine hohe Spannkraft erreicht, so daß eine hohe Kompression der Knochenfragmente erzielbar ist.

Die eigentliche Druckplatte kann so ausgebildet sein, daß dann, wenn der Spannblock nicht benötigt wird, die herkömmliche Spannweise mit Spannlöchern, die eine 27°- oder 45°-Neigung aufweisen, möglich ist. Auch kann an die eigentliche Druckplatte ohne Schwierigkeiten der handelsübliche Plattenspanner angesetzt werden, falls dies erforderlich sein sollte.

Durch die erfindungsgemäße Vorrichtung wird keine zusätzliche Plattenverteuerung bedingt, sondern die Hilfsvorrichtungen, die erforderlich sind, um den eigentlichen Spannblock an der Platte festzulegen, sind einfach ausgebildet und daher kostengünstig herzustellen. Ein Beschädigen der Platte tritt durch die erzeugte Kompressionswirkung nicht ein, sondern, falls solche Beschädigungen eintreten, erfolgen diese nur im Spannblock.

Es ist vorgesehen, daß die ortsfeste Festlegung des eigentlichen Spannblockes an der Druckplatte über eine Schraube erfolgt, die mit einem Gewinde ausgerüstet ist, das mit einem Innengewinde einer Bohrung in der Druckplatte kämmt. Bei dieser Ausführungsform stützt sich die Spannvorrichtung an der Druckplatte ab und die Vorrichtung zur Erzeugung der Vorschubbewegung des Knochenfragmentes ist eine schiefe Ebene, die mit dieser Abstützung so zusammenwirkt, daß ein Vorschieben des Knochenfragmentes erfolgt, ohne daß die den Spannblock mit dem Knochenfragment verbindende Schraube gleichzeitig eine in Schraubenlängsachse gerichtete Zugkraft aufnehmen muß. Hierdurch wird erreicht, daß einerseits die Schraube nicht abknicken kann, andererseits der Knochen weniger belastet wird und z. B. auch bei relativ spröden oder dünnen Knochen eine solche Druckosteosynthese möglich ist.

Ausführungsbeispiele der Erfindung werden nachfolgend anhand der Zeichnungen erläutert. Die Zeichungen zeigen dabei in

Fig. 1 eine Ausführungsform der Erfindung, wobei der Spannblock an der Druckplatte festgeschraubt wird, in

Fig. 2 die Einrichtung gemäß Fig. 1 in der auf der Druckplatte festgelegten Stellung, in

Fig. 3 in einer auseinandergezogenen Darstellungsweise eine abgeänderte Ausführungsform, bei der die druckerzeugende Vorrichtung durch zwei miteinander zusammenwirkende schiefe Ebenen gebildet wird, in

Fig. 4 die Einrichtung gemäß Fig. 3 im eingebauten Zustand, in

Fig. 5 in einer auseinandergezogenen Darstellungsweise eine abgeänderte Ausführungsform der druckerzeugenden Vorrichtung, wobei in diesem Fall die schiefe Ebene horizontal ausgerichtet ist, in

Fig. 6 die Einrichtung gemäß Fig. 5 im eingebauten Zustand, in

Fig. 7 die Einrichtung gemäß Fig. 5 in einer wirksamen Druckstellung und in

Fig. 8 die zur Wirkstellung gemäß Fig. 7 zugehörige Stellung der beiden Knochenfragmente.

In der nachfolgenden Figurenbeschreibung sind so weit wie möglich gleiche Teile mit gleichen Bezugszeichen versehen und nur die unterschiedlich ausgebildeten Teile mit abgeänderten Bezugszeichen benannt.

In den Zeichnungen ist mit 1 eine Druckplatte bezeichnet, die eine sogenannte Fixationsseite 2 und eine Spannseite 3 aufweist. In der Fixationsseite sind bei dem dargestellten Ausführungsbeispiel drei Löcher 4 vorgesehen, die der Aufnahme von Befestigungsschrauben 5 handelsüblicher Art dienen.

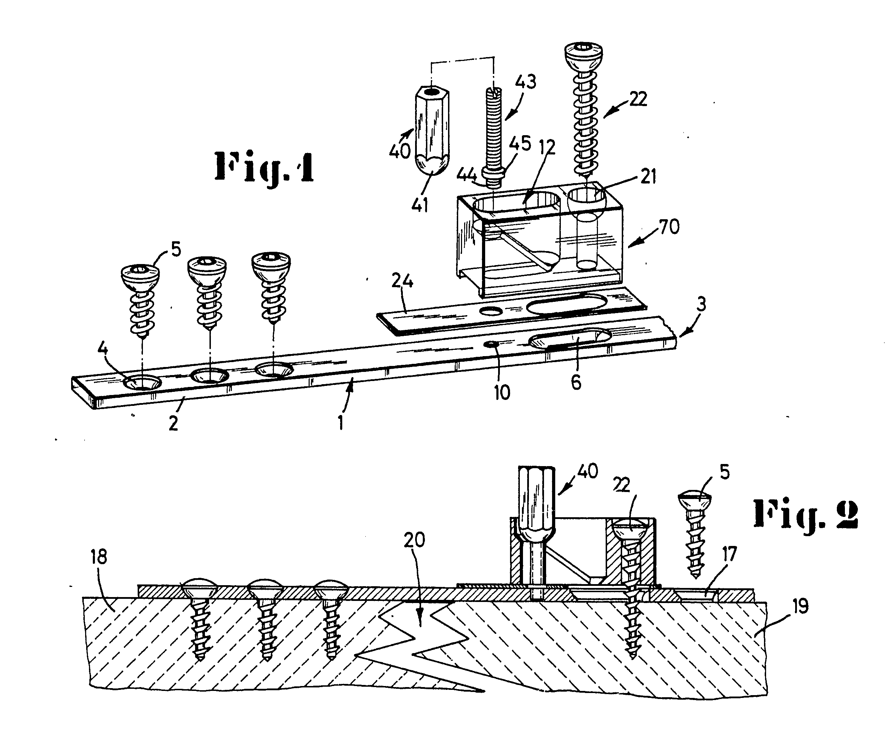

In der Spannseite 3 ist in der Druckplatte 1 ein Langloch 6 vorgesehen, über das gemäß Fig. 1 ein Spannblock 70 gesetzt werden kann. Innerhalb des Spannblockes 70 ist ein Schraubenloch 12 vorgesehen, das eine ovale Randneigung von etwa 45° aufweist. Mit der Randneigung des Schraubloches 12 arbeitet ein als Spannmutter 40 ausgebildetes Spannmittel zusammen, dessen Kopf 41 eine entsprechende an sich bekannte sphärische Geometrie aufweist.

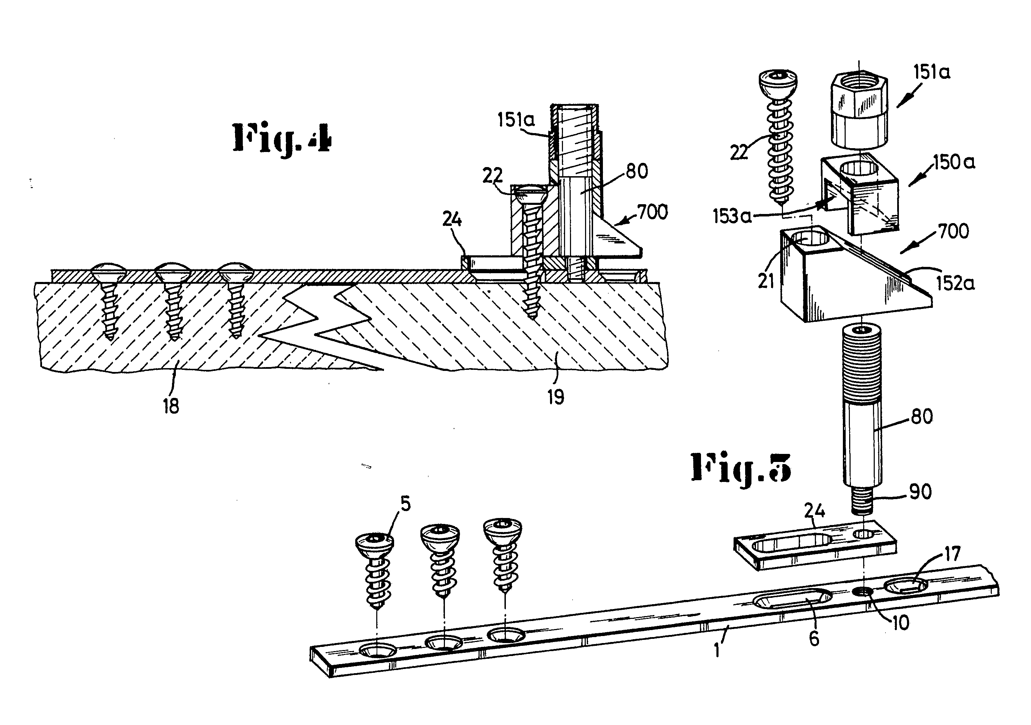

Der Spannblock 70 weist eine Aufnahmeöffnung 21 auf, die eine Knochenschraube 22 aufnimmt, die durch das Langloch 6 greift und sich in den Knochen 19 einschraubt. Fig. 2 zeigt, daß die Knochenschraube 22 auf einem großen Teil ihrer Länge innerhalb der Aufnahmeöffnung 21 geführt und gehalten ist.

Ein als Schraube ausgebildetes Gegenlager 43 weist ein Außengewinde 44 auf, das nach oben durch eine Widerlagerscheibe 45 begrenzt wird, die sich bei eingeschraubtem Gewinde 44 in eine Bohrung 10 auf der Oberseite der Druckplatte 1 aufliegt. Im oberen Bereich der Schraube 43 ist ein weiteres Gewinde vorgesehen, das mit dem Innengewinde der Spannmutter 40 kämmt, so daß die Spannmutter 40 auf der Schraube 43 nach unten bewegt werden kann. Hierbei kommt die Spannmutter 40 mit der sphärischen Geometrie ihres Kopfes 41 zur Anlage an der schiefen Ebene in dem Schraubloch 12.

Zwischen der Oberseite der Druckplatte 1 und der Unterseite des Spannblockes 70 befindet sich eine Zwischenlage 24, auf der sich der Spannblock 70 ohne Berührung mit der Druckplatte 1 bei seiner Verschiebebewegung bewegen kann.

Zusätzlich sind auf der Spannseite der Druckplatte 1 weitere Befestigungslöcher vorgesehen, wobei in Fig. 2 das Befestigungsloch 17 erkennbar ist. Mit diesem Befestigungsloch 17 kann eine Befestigungsschraube 5 zusammenwirken. In den Zeichnungen sind mit 18 und 19 zwei Knochenfragmente bezeichnet, die im Bereich 20 gebrochen sind und in einem Abstand voneinander stehen, wobei dieser Bereich 20 durch die eigentliche Druckplatte wieder zusammengeführt werden soll.

Die Wirkungsweise dieser Einrichtung ist wie folgt: Der Spannblock 70 wird über die Schraube 43 unter Zwischenschaltung der Zwischenlage 24 auf der Oberseite der Druckplatte 1 oberhalb des Langloches 6 festgelegt. Anschließend wird die Knochenschraube 22 durch die Aufnahmeöffnung 21 hindurchgeführt und in den Knochenteil 19 eingeschraubt. Dabei befindet sich die Knochenschraube 22 an dem äußeren rechten Rand des Langloches 6. Durch Aufschrauben der Spannmutter 40 auf das obere Teil des Gewindes der Schraube 43 schraubt sich die Spannmutter 40 in Verbindung mit der im Schraubenloch 12 vorgesehenen schiefen Ebene nach unten und drückt dabei den Spannblock 70 und somit das Knochenfragment 19 in der Zeichnung gemäß Fig. 1 nach links. Dadurch werden die Knochenfragmente 18 und 19 aufeinanderzu bewegt, ohne daß die für diese Schubbewegung erforderliche Kraft als Zugkraft im Knochenteil 19 aufgenommen werden muß, sondern die Schraube 22 hat lediglich die Aufgabe, den Knochen zu schieben.

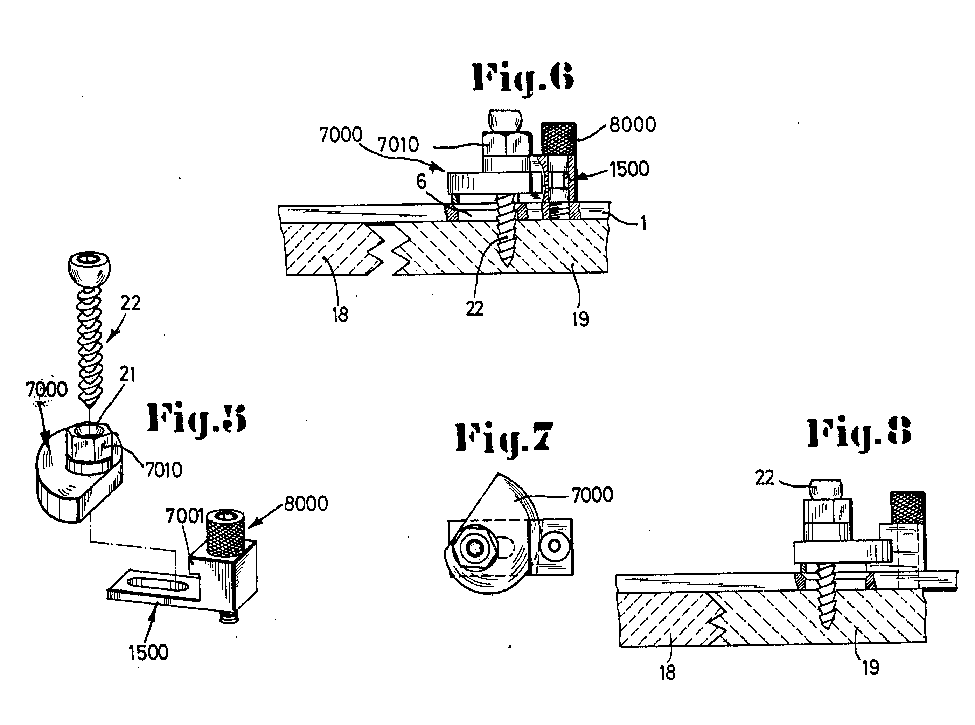

Bei der Ausführungsfrom gemäß den Fig. 3 und 4 ist ebenfalls eine Zwischenlage 24 vorgesehen. Der eigentliche Spannblock 700 weist eine schiefe Ebene 152a auf, die mit einer schiefen Ebene 153a eines Bauteiles 150a zusammenwirkt. In der Bohrung 10 der Druckplatte 1 wird eine Schraube 80 unter Zuhilfenahme des Gewindes 90 festgelegt, wobei sich auf diese Schraube 80 eine Mutter 151a aufschraubt. In dem Spannblock 700 ist die Aufnahmeöffnung 21 vorgesehen, die der Aufnahme der Knochenschraube 22 dient. Bei dieser Ausführungsform erfolgt das Aufbringen des erforderlichen Spanndruckes in der schon anhand der Fig. 1 und 2 erläuterten Weise.

Bei der in den Fig. 5 bis 8 dargestellten Ausführungsform wird zum Aufbringen des erforderlichen Spanndruckes eine schiefe Ebene benutzt, die horizontal ausgerichtet ist, d. h. es wird ein Spannblock 7000 eingesetzt, der als spiralförmige Exzenterscheibe ausgebildet ist. Die Scheibe 7000 ist an einem Spannmittel 1500 festgelegt, das über eine als Gegenlager 8000 ausgebildete Schraube an der Druckplatte 1 befestigt ist. Das Spannmittel 1500 weist eine Schulter 7001 auf, die die spiralförmige Scheibe 7000 übergreift. Durch die spiralförmige Scheibe 7000 verläuft die die Knochenschraube 22 aufnehmende Aufnahmeöffnung 21 und das Aufringen des erforderlichen Druckes erfolgt durch Drehen der spiralförmigen Scheibe 7000 unter Zuhilfenahme einer entsprechenden Mutter 7010. Auch bei dieser Ausführungsform erfolgt die Spannbewegung entsprechend der anhand der Fig. 1 und 2 erläuterten Weise.

**Patentansprüche**

1. Spannvorrichtung für eine Druckplatte (1) zur Durchführung einer Druckosteosynthese mit einem auf der Oberseite der Druckplatte (1) über einem in der Druckplatte (1) vorgesehenen Langloch (6) sich abnehmbar abstützenden längs der Druckplatte verschiebbaren Spannblock (70, 700, 7000), einem gegenüber der Druckplatte (1) in Spannrichtung ortsfesten Gegenlager (43, 80, 8000) sowie einem mit dem Gegenlager zusammenwirkenden Spannmittel (40, 150a, 1500), wenigstens einem vom Spannblock nicht abgedeckten Befestigungsloch (17) in der Druckplatte (1) auf deren Spannseite (3) und mit einem den Spannblock durchsetzenden und diesen mit dem zugeordneten Knochenfragment (19) verbindenden Verbindungsmittel, dadurch gekennzeichnet, daß das Verbindungsmittel als Knochenschraube (22) ausgebildet ist, die den Spannblock (70, 700, 7000) in Richtung senkrecht zur Ebene der Druckplatte (1) festlegt, und daß das die Spannbewegung bewirkende Spannmittel (40, 150a, 1500) mit einer schiefen Ebene des Spannblocks (70, 700, 7000) zusammenwirkt und zumindest in Richtung parallel zur Ebene der Druckplatte (1) ortsfest mit dem in die Druckplatte einschraubbaren Gegenlager (43, 80, 8000) verbunden ist.

2. Spannvorrichtung nach Anspruch 1, dadurch gekennzeichnet, daß die Spannbewegung entlang der schiefen Ebene durch das Zusammenwirken der sphärischen Geometrie des Kopfes (41) einer Spannmutter (40) und eines ovalen, geneigten und mit einer Randneigung ausgerüsteten Schraubenloches (12) im Spannblock (70) erzielt wird (Fig. 1 und 2).

3. Spannvorrichtung nach Anspruch 1, dadurch gekennzeichnet, daß die schiefe Ebene durch zwei gegeneinander verschiebbare, zusammenwirkende geneigte Flächen (152a und 153a) gebildet ist (Fig. 3 und 4).

4. Spannvorrichtung nach Anspruch 1 bis 3, dadurch gekennzeichnet, daß zwischen Spannblock (70, 700) und Druckplatte (1) eine Zwischenlage (24) eingeschaltet ist (Fig. 1 bis 4).

5. Spannvorrichtung nach Anspruch 1, dadurch gekennzeichnet, daß die schiefe Ebene des Spannblockes (7000) durch eine am Umfang spiralförmige Exzenterscheibe gebildet ist.

6. Spannvorrichtung nach Anspruch 5, gekennzeichnet durch einen an dem sich ortsfest an der Druckplatte (1) abstützenden Spannmittel (1500) angeordneten Schultervorsprung (7001), der die Exzenterscheibe des Spannblockes (7000) übergreift.

7. Spannvorrichtung nach einem der Ansprüche 1 bis 6, dadurch gekennzeichnet, daß die die Knochenschraube (22) aufnehmende Aufnahmeöffnung (21) im Spannblock (70, 700, 7000) die Knochenschraube (22) auf einem großen Teil ihrer Länge führt.

## Claims

1. Clamping device for a pressure plate (1) for carrying out pressure osteosynthesis, with a clamping block (70, 700, 7000) which is supported removably on the top side of the pressure plate (1) via a slot (6) provided in the pressure plate (1) and which is displaceable along the pressure plate, with an abutment (43, 80, 8000) fixed in place in the clamping direction relative to the pressure plate (1) and with a clamping means (40, 150a, 1500) interacting with the abutment, with at least one fastening hole (17), not covered by the clamping block in the pressure plate (1) on its clamping side (3), and with a connecting means passing through the clamping block and connecting this to the associated bone fragment (19), characterised in that the connecting means is designed as a bone crew (22) which fixes the clamping block (70, 700, 7000) in a direction perpendicular to the plane of the pressure plate (1), and in that the clamping means (40, 150a, 1500) causing the clamping movement interacts with an inclined plate of the clamping block (70, 700, 7000) and is connected, at least in a direction parallel to the plane of the pressure plate (1), fixedly to the abutment (43, 80, 8000) which can be screwed into the pressure plate.

2. Clamping device according to claim 1, characterised in that the clamping movement along the inclined plane is achieved as result of the interaction between the spherical geometry of the head (41) of a clamping nut (40) and an oval inclined screw hole (12) provided with an edge slope, in the clamping block (70) (Figures 1 and 2).

3. Clamping device according to claim 1, characterised in that the inclined plane is formed by two interacting sloping faces (152a and 153a) displaceable relative to one another (Figures 3 and 4).

4. Clamping device according to claims 1 to 3, characterised in that a shim (24) is inserted between the clamping block (70, 700) and pressure plate (1) (Figures 1 to 4).

5. Clamping device according to claim 1, characterised in that the the inclined plane of the clamping block (7000) is formed by a cam disc spiralshaped on its periphery.

6. Clamping device according to claim 5, characterised by a shoulder projection (7001) which is arranged on the clamping means (1500) supported fixedly on the pressure plate (1) and which engages over the cam disc of the clamping block (7000).

7. Clamping device according to one of claims 1 to 6, characterised in that the receiving orifice (21) in the clamping block (70, 700, 7000), which receives the bone screw (22), guides the bone screw (22) over a large part of its length.

## Revendications

1. Dispositif de contention d'une plaque de coaptation pour procéder à une ostéosynthèse par coaptation comprenant un bloc tendeur (70, 700, 7000) amovible appliqué sur la face supérieure de la plaque de coaptation (1) au-dessus d'un trou oblong pratiqué dans la plaque (1) et coulissable le long de la plaque, une contre-butée (43, 80, 8000) stationnaire suivant la direction de tension par rapport à la plaque (1) ainsi qu'un moyen contentif (40, 150a, 1500) coopérant avec la contre-butée, au moins un trou de fication (17) non recouvert par le bloc tendeur prévu dans la plaque (1) sur son côté tendeur (3) et un moyen de liaison intimement lié au bloc tendeur pour relier celui-ci au fragment osseux associé (19), caractérisé en ce que le moyen de liaison est conformé en une vis pour os (22) qui immobilise le bloc tendeur (70, 700, 7000) en direction verticale par rapport au plan de la plaque de pression (1), et en ce que le moyen contentif (40, 150a, 1500) provoquant le déplacement sous tension coopère avec un plan oblique de bloc tendeur (70, 700, 7000) et est relié stationnairement au moins en direction parallèle au plan de la plaque de coaptation (1), à la contre-butée (43, 80, 8000) vissée dans la plaque de coaptation.

2. Dispositif de contention selon la revendication 1, caractérisé en ce que le déplacement sous tension le long du plan oblique est obtenu par la coopération de la géométrie sphérique de la tête (41) d'un écrou tendeur (40) et d'un trou oblong pour vis (12) incliné et pourvu d'une bordure inclinée dans le bloc-tendeur (70).

3. Dispositif de contention selon la revendication 1, caractérisé en ce que le plan oblique est formé par deux surfaces (152a et 153a) inclinées, coopérantes et coulissables l'une contre l'autre (Figs. 3 et 4).

4. Dispositif de contention selon l'une des revendications 1 à 3, caractérisé en ce qu'entre le bloc tendeur (70, 700) et la plaque de coaptation (1) est insérée une plaquette intermédiaire (24) (Figs. 1 à 4).

5. Dispositif de contention selon la revendication 1, caractérisé en ce que le plan oblique du bloc tendeur (7000) est formé par un excentrique à profil en spirale.

6. Dispositif de contention selon la revendication 5, caractérisé en ce qu'un épaulement saillant (7001) sur le moyen contentif (1500) reposant fixement sur la plaque de coaptation (1) surplombe l'excentrique du bloc tendeur (7000).

7. Dispositif de contention selon l'une des revendications 1 à 6, caractérisé en ce que l'orifice (21) recevant la vis pour os (22) dans le bloc tendeur (70, 700, 7000) guide la vis pour os (22) sur une grande partie de sa longueur.

Fig. 1

Fig. 2

# Fig.4

# Fig.5

151a

22

80

700

24

18

19

151a

22

150a

153a

700

21

152a

80

90

5

24

17

6

10

1

0 016 338

Fig.6

Fig.8

Fig.7

Fig.5